# EUROPÄISCHE PATENTSCHRIFT

(11) **EP 1 238 274 B1**
(45) Veröffentlichungstag und Bekanntmachung des Hinweises auf die Patenterteilung: **16.06.2004**
(21) Anmeldenummer: 00984870.6
(22) Anmeldetag: 26.10.2000
(51) Int. Cl.: G01N 33/48

(54) **TESTELEMENT-ANALYSESYSTEM MIT INFRAROTDETEKTOR**
TEST ELEMENT ANALYSIS SYSTEM WITH INFRARED DETECTOR
SYSTEME D'ANALYSE POUR ELEMENT D'ESSAI, DOTE D'UN DETECTEUR INFRAROUGE

(30) Priorität: 29.10.1999 DE 19952215
(43) Veröffentlichungstag der Anmeldung: 11.09.2002
(73) Patentinhaber: Roche Diagnostics GmbH, 68298 Mannheim (DE)
(72) Erfinder: HAAR, Hans-Peter, 69168 Wiesloch (DE)
(74) Vertreter: Pfeifer, Hans-Peter, Dr.
(86) Internationale Anmeldenummer: PCT/DE2000/003804
(87) Internationale Veröffentlichungsnummer: WO 2001/033214

(56) Entgegenhaltungen:
- EP-A- 0 801 926
- EP-A- 0 851 229
- EP-A- 0 943 912
- US-A- 5 578 499
- US-A- 5 820 264

## Beschreibung

Die Erfindung betrifft ein Testelement-Analysesystem zur analytischen Untersuchung einer Probe, insbesondere einer Körperflüssigkeit von Menschen oder Tieren. Zu dem System gehören zwei Bestandteile, nämlich Testelemente, die eine Meßzone aufweisen, in die die zu untersuchende Probe zur Durchführung einer Analyse gebracht wird, um eine für die Analyse charakteristische Meßgröße zu messen, und ein Auswertegerät mit einer Testelementhalterung, um ein Testelement in einer Meßposition zur Durchführung der Messung zu positionieren und einer Meß- und Auswerteelektronik zur Messung der charakteristischen Meßgröße und Ermittlung eines hierauf basierenden Analyseresultates.

Testelement-Analysesysteme sind insbesondere in der Medizin für die Analyse von Urin und Blut gebräuchlich. Die Testelemente haben meist die Form von Teststreifen, jedoch sind auch andere Formen von Testelementen, beispielsweise flache, näherungsweise quadratische Plättchen, gebräuchlich.

In der Regel enthalten die Testelemente Reagenzien, deren Reaktion mit der Probe zu einer physikalisch nachweisbaren Veränderung des Testelementes führt, die mit dem zu dem System gehörigen Auswertegerät gemessen wird. Gebräuchlich sind insbesondere photometrische Analysesysteme, bei denen die Reaktion eine Farbänderung,in einer Nachweisschicht des Testelementes verursacht, die photometrisch gemessen wird. Daneben haben elektrochemische Analysesysteme eine erhebliche Bedeutung, bei denen infolge der Reaktion eine als Spannung oder Stromfluß meßbare elektrochemische Veränderung des Testelementes stattfindet. Neben diesen mit Reagenzien arbeitenden Analysesystemen werden auch reagenzfreie Analysesysteme diskutiert, bei denen nach Kontaktierung des Testelementes mit der Probe eine analytisch charakteristische Eigenschaft der Probe selbst (beispielsweise deren optisches Absorptionsspektrum) gemessen wird. Die Erfindung ist grundsätzlich für alle diese Verfahren verwendbar.

Teilweise werden Testelement-Analysesysteme in medizinischen Labors eingesetzt. Die Erfindung richtet sich jedoch insbesondere auf Anwendungsfälle, bei denen die Analyse durch den Patienten selbst durchgeführt wird, um seinen Gesundheitszustand laufend zu überwachen ("home-monitoring"). Von besonderer medizinischer Bedeutung ist dies für die Behandlung von Diabetikern, die die Konzentration von Glucose im Blut mehrfach täglich bestimmen müssen, um ihre Insulininjektionen danach einzustellen. Für derartige Zwecke müssen die Auswertegeräte leicht und klein, batteriebetrieben und robust sein.

Ein grundlegendes Problem besteht darin, daß die für die Analyse charakteristische Meßgröße meist stark temperaturabhängig ist. Diese Temperaturabhängigkeit liegt häufig bei ein bis zwei Prozent pro Grad. Im Bereich des home-monitoring ist es unvermeidlich, daß das Analysesystem starken Temperaturänderungen ausgesetzt ist. Dort muß mit Schwankungen der Temperatur von mindestens ± 5° gerechnet werden, wobei wesentlich höhere Temperaturschwankungen vorkommen können, wenn die Messung auch unter ungewöhnlichen Bedingungen (beispielsweise im Auto oder im Freien) möglich sein soll.

Um die daraus resultierenden Meßungenauigkeiten zu vermeiden, wurde vorgeschlagen, die Meßzone des Testelementes mittels einer entsprechenden Temperierungseinrichtung auf eine bestimmte konstante Temperatur zu temperieren. Beispielsweise ist in dem US-Patent 5,035,862 die Temperierung individueller Testfelder von Urinteststreifen mittels induktiver Beheizung beschrieben. Ein anderes Beispiel bei einem Blutanalysegerät zeigt die DE 3321783 A1. Derartige Verfahren sind jedoch bei kleinen batteriebetriebenen Geräten wegen des hohen Energieverbrauches unpraktikabel.

Bei manchen Analysesystemen wird die Temperatur während der Messung in dem Gehäuse des Auswertegerätes elektrisch (mittels eines Thermoelementes oder Thermowiderstandes) bestimmt und die so gemessene Temperatur bei der Ermittlung des Analyseresultates berücksichtigt. Ein Beispiel zeigt die WO 99/06822. Eine solche Korrektur kann genau sein, wenn sich die Temperatur in der Umgebung des Auswertegerätes und des Testelementes vor der Messung längere Zeit nicht geändert hat und deswegen die tatsächliche Temperatur der Probe in der Meßposition mit der elektrisch gemessenen Temperatur gut übereinstimmt. Insbesondere im Bereich des home-monitoring ist diese Bedingung vielfach jedoch nicht erfüllt, weil die Lebensumstände des Patienten es erfordern, daß er Analysen an verschiedenen Orten und unter wechselnden Temperaturbedingungen durchführt.

Zur Lösung dieses Problems wird in dem US-Patent 5,405,511 vorgeschlagen, die Temperatur wiederholt in regelmäßigen Abständen zu messen und die Korrekturtemperatur durch Extrapolation auf Basis des über einen gewissen Zeitraum gemessenen Temperaturverlaufs zu bestimmen. Dies erfordert allerdings, daß die Temperatur vor der Analyse über einige Minuten fortlaufend oder in bestimmten Abständen bestimmt wird. Um die damit verbundene Wartezeit vor Durchführung des Tests zu vermeiden, werden gemäß dem US-Patent 5,405,511 auch dann Temperaturmessungen im Abstand von einigen Minuten durchgeführt, wenn das Gerät ausgeschaltet ist. Dadurch kann die Extrapolation auf die Korrekturtemperatur unmittelbar nach dem Einschalten des Gerätes durchgeführt werden. Mit diesem Verfahren ist jedoch ein erhöhter Energieverbrauch verbunden, weil die Geräteelektronik jeweils im Abstand von wenigen Minuten zur Bestimmung der Temperatur in Betrieb gesetzt werden muß. Außerdem ist die Abschätzung der Korrekturtemperatur mittels eines Extrapolationsalgorithmus nicht unter allen Betriebsbedingungen zuverlässig.

In der EP 0851229 A1 ist ein Analysesystem beschrieben, bei dem an der Halterung des Testelementes in dem Auswertegerät oder an dem Testelement selbst eine Temperaturmeßfläche vorgesehen ist, die mit einem thermochromen Flüssigkristall (TLC) beschichtet ist. Die Temperatur des TLC wird durch photometrische Messung ermittelt. Eine gute Übereinstimmung dieser Messung mit der tatsächlichen Temperatur der Meßzone wird dabei nur dann erreicht, wenn das Testelement selbst mit dem TLC beschichtet ist. Dies führt jedoch zu erheblichen zusätzlichen Kosten bei der Herstellung der Testelemente. Außerdem läßt sich eine akzeptable Genauigkeit der Temperaturmessung nur mit einem hohen meßtechnischen Aufwand erreichen.

EP-A-0 943 912 und US-A-5 578 499 beschreiben Vorrichtungen zum Nachweis von Verbindungen auf Oberflächen, in denen ein Infrarotdetektor eingesetzt wird). US-A-5 578 499 beschreibt zusätzlich IR-Strahlen-Transportmittel. Der IR-Detektor dient jedoch nicht zur Messung der Temperatur der Oberfläche.

EP-A-0 801 926 und US-A-5 820 264 befassen sich mit klinischen Thermometern, bei denen die Körpertemperatur durch eine Infrarot-Messung der von dem Trommelfell ausgehenden Wärmestrahlung bestimmt wird.

Der Erfindung liegt die Aufgabe zugrunde, ein Testelement-Analysesystem zur Verfügung zu stellen, mit dem durch eine verbesserte Temperaturkompensation eine erhöhte Meßgenauigkeit erreicht wird. Dies soll mit einem geringen Aufwand, der für home-monitoring-Systeme vertretbar ist, möglich sein.

Die Aufgabe wird bei einem Testelement-Analysesystem der vorstehend erläuterten Art dadurch gelöst, daß das Auswertegerät zur Bestimmung der im Bereich der Meßzone des Testelementes herrschenden Temperatur einen Infrarotdetektor aufweist.

Die speziellen Anforderungen üblicher Teststreifen-Analysesysteme bringen es mit sich, daß es in den meisten Fällen nicht möglich ist, einen Infrarotdetektor so zu positionieren, daß er unmittelbar die von der Meßzone kommende IR-Strahlung hinreichend selektiv und empfindlich detektiert, um die erforderliche Genauigkeit der Temperaturmessung zu gewährleisten. Im Rahmen der Erfindung ist zur Lösung dieses Problems vorzugsweise vorgesehen, daß die Meßzone und der Infrarotdetektor durch ortsselektive Infrarotstrahlen-Transportmittel miteinander verbunden sind, die folgende Anforderungen erfüllen:
- Sie führen dem Detektor selektiv die von der Meßzone kommende IR-Strahlung zu.
- Ein sehr hoher Anteil der von der Meßzone ausgehenden IR-Strahlung gelangt zu dem Detektor, d.h. die Transportmittel arbeiten weitgehend verlustfrei.

Diese Anforderungen können im Prinzip mit Hilfe eines optischen Ausbildungssystems erfüllt werden, das mindestens eine Linse aufweist. Wesentlich bevorzugte Bestandteile der Infrarotstrahlen-Transportmittel sind jedoch ein Hohlleiter mit für Infrarotstrahlen reflektierender Innenwand, insbesondere aus metallisiertem Kunststoff, und/oder ein innerhalb des Gehäuses angeordneter Abbildungsspiegel. Diese Elemente ermöglichen einen nahezu verlustfreien Transport der IR-Strahlung von der Meßzone zu dem Infrarotdetektor und zugleich eine sehr gute Selektivität. Dabei sind die Kosten gering und es ist ohne Probleme möglich, einen gekrümmt oder mehreckig verlaufenden (nichtgeraden) Strahlengang zwischen der Meßzone und dem Infrarotdetektor zu realisieren. Dadurch ist eine auf die Bedürfnisse eines Testelement-Analysesystems optimal abgestimmte Realisierung der Infrarot-Temperaturmessung der Meßzone möglich.

Die Erfindung wird nachfolgend anhand von in den Figuren dargestellten Ausführungsbeispielen näher erläutert. Die darin beschriebenen Besonderheiten können einzeln oder in Kombination miteinander eingesetzt werden, um bevorzugte Ausgestaltungen der Erfindung zu schaffen. Es zeigen:
- Fig. 1: Ein erfindungsgemäßes Testelement-Analysesystem in perspektivischer Darstellung,
- Fig. 2: eine Teil-Schnittdarstellung eines erfindungsgemäßen Analysesystems,
- Fig. 3: eine Teil-Schnittdarstellung einer alternativen Ausführungsform,
- Fig. 4: eine Schnitt-Prinzipskizze einer weiteren alternativen Ausführungsform,
- Fig. 5: eine Schnitt-Prinzipskizze einer dritten alternativen Ausführungsform.

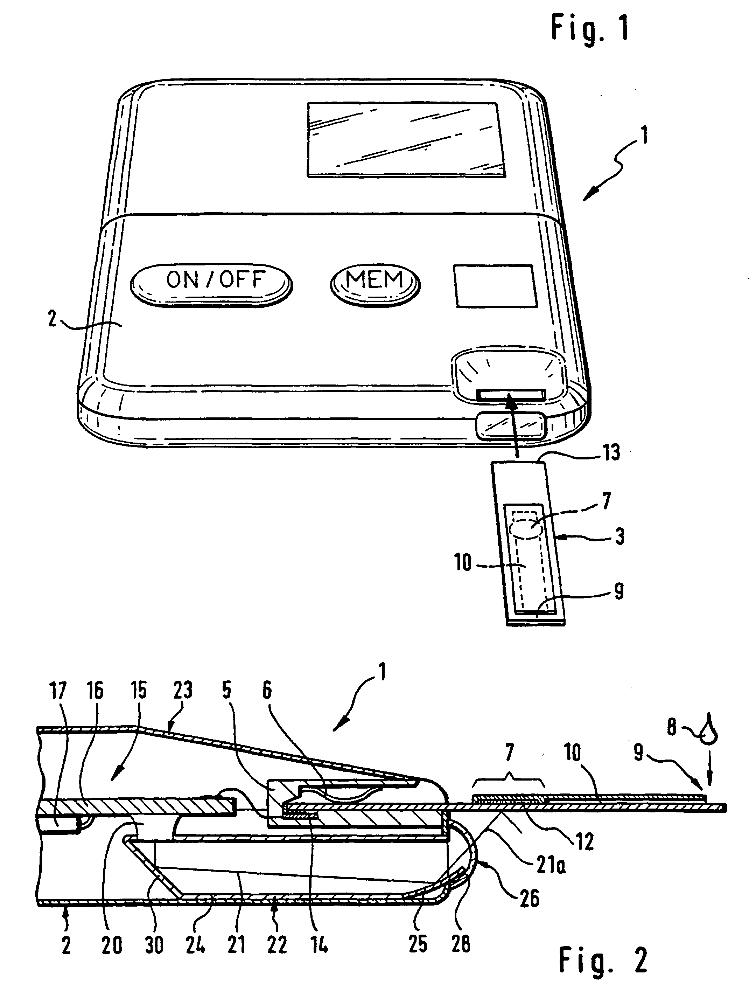

Das in den Figuren 1 und 2 dargestellte Analysesystem 1 besteht aus einem Auswertegerät 2 und zur einmaligen Verwendung vorgesehenen (disposiblen) Testelementen 3.

Das Auswertegerät 2 hat eine Testelementhalterung 5, mit der ein Testelement 3 in der in Figur 2 dargestellten Meßposition positioniert wird. Das Testelement 3 ist durch geeignete Mittel, beispielsweise eine Blattfeder 6, in der Meßposition fixiert.

Zur Durchführung einer Messung wird die Probenflüssigkeit (beispielsweise Blut) in eine Meßzone 7 gebracht. Bei der dargestellten Ausführungsform eines Testelementes 3 geschieht dies dadurch, daß ein Blutstropfen 8 auf eine am Ende des Testelementes 3 vorgesehene Probenauftragszone 9 aufgebracht und von dort in einem Kapillarspalt 10 zu der Meßzone 7 gesaugt wird. In der Meßzone 7 befindet sich eine Reagenzschicht 12, die von der Probenflüssigkeit aufgelöst wird und mit deren Bestandteilen reagiert.

Die Reaktion führt zu einer meßbaren Veränderung in der Meßzone 7. Im dargestellten Fall eines elektrochemischen Testelementes erfolgt die Messung einer elektrischen Meßgröße mittels in der Meßzone vorgesehener, in den Figuren nicht dargestellter Elektroden, die mit Kontaktstreifen 13 verbunden sind. Die Kontaktstreifen 13 stellen in der Meßposition einen elektrischen Kontakt zu entsprechenden Gegenkontakten 14 der Testelementhalterung 5 her, die mit einer Meß- und Auswerteelektronik 15 verbunden sind. Im Hinblick auf eine möglichst kompakte Bauweise und hohe Zuverlässigkeit ist die Meß- und Auswerteelektronik 15 hochintegriert. Im dargestellten Fall besteht sie im wesentlichen aus einer Leiterplatine 16 und einem Spezial-IC (ASIC) 17.

Auf der Leiterplatine 16 ist auch ein Infrarotdetektor 20 zur Bestimmung der im Bereich der Meßzone 7 herrschenden Temperatur montiert. Geeignete Infrarotdetektoren sind kostengünstig verfügbar. Vorzugsweise wird ein Detektortyp gewählt, der zur Eigenkalibration einen integrierten Temperatursensor (z.B. ein NTC-Halbleiterelement) einschließt.

Generell ist es vorteilhaft, wenn der Infrarotdetektor 20 in dem Sinne in die Meß- und Auswerteelektronik 15 integriert ist, daß eine starre mechanische Verbindung zwischen dem Infrarotdetektor 20 und den übrigen Bestandteilen der Meß- und Auswerteelektronik 15 besteht. Durch kurze und mechanisch starre Leiterverbindungen zwischen dem Infrarotdetektor 20 und den übrigen Bestandteilen der Meß- und Auswerteelektronik 15 wird nicht nur eine kompakte Bauweise, sondern vor allem eine hohe mechanische und elektronische Stabilität sowie eine gute langfristige Zuverlässigkeit erreicht.

Nachteilig erscheint dabei zunächst, daß der in Figur 2 gestrichelt eingezeichnete Übertragungsweg 21, den die IR-Strahlung von der Meßzone 7 zu dem Infrarotdetektor 20 zurücklegen muß, relativ lang und nicht gerade ist. Dies gilt insbesondere, wenn das Auswertegerät die in der Praxis (im Hinblick auf eine einfache Handhabung) gewünschte sehr flache Bauform hat und infolgedessen die Testelementhalterung 5 und die Elektronikeinheit 15 nicht übereinander angeordnet werden können.

Besondere zusätzliche Probleme ergeben sich, wenn das Testelement und die Halterung des Auswertegerätes - wie dargestellt - so ausgebildet sind, daß das Testelement 3 in der Meßposition aus dem Gehäuse 23 des Auswertegerätes 2 herausragt. Diese Bauweise ist für die Handhabung des Analysesystems vorteilhaft, weil die Probe in die Meßzone 7 gebracht werden kann, während sich das Testelement in der Meßposition befindet. Für die Bestimmung der im Bereich der Meßzone 7 herrschenden Temperatur ist damit jedoch der Nachteil verbunden, daß der Übertragungsweg 21 durch ein in dem Gehäuse 2 vorgesehenes Fenster 26 verlaufen muß und einen außerhalb des Gehäuses 23 verlaufenden Abschnitt 21a einschließt.

Die insgesamt mit 22 bezeichneten Infrarotstrahlen-Transportmittel ermöglichen auch in derartig problematischen Fällen eine selektive und empfindliche Erfassung der von der Meßzone 7 ausgehenden Infrarotstrahlung. Im dargestellten Fall bestehen sie aus einem Hohlleiter 24 mit für Infrarotstrahlen reflektierender Innenwand und einem innerhalb des Gehäuses 23 des Auswertegerätes 2 angeordneten Abbildungsspiegel 25.

Der Hohlleiter 24 ist als zumindest auf seiner Innenseite metallisiertes (insbesondere vergoldetes) Kunststoffteil realisiert. Mittels eines solchen Hohlleiters 24 kann auf einfache und kostengünstige Weise der gewünschte Übertragungsweg 21 für die IR-Strahlen innerhalb des Gehäuses 25 realisiert werden.

Soweit - wie bei dem dargestellten Testelement-Analysesystem - der Übertragungsweg 21 der IR-Strahlen auch einen außerhalb des Gehäuses 25 des Auswertegerätes 2 verlaufenden Abschnitt 21a aufweist, ist es vorteilhaft, wenn auf diesem Abschnitt die erforderliche selektive Erfassung der aus der Meßzone 7 kommenden IR-Strahlung mittels eines optischen Abbildungssystems realisiert wird, wobei die in Figur 2 dargestellte Verwendung eines konkav gekrümmten Abbildungsspiegels 25 bevorzugt ist. Das optische Fenster 26 ist vorzugsweise mittels einer für Infrarotstrahlen durchlässigen Scheibe 28, insbesondere einer Polyethylenfolie, staubdicht verschlossen.

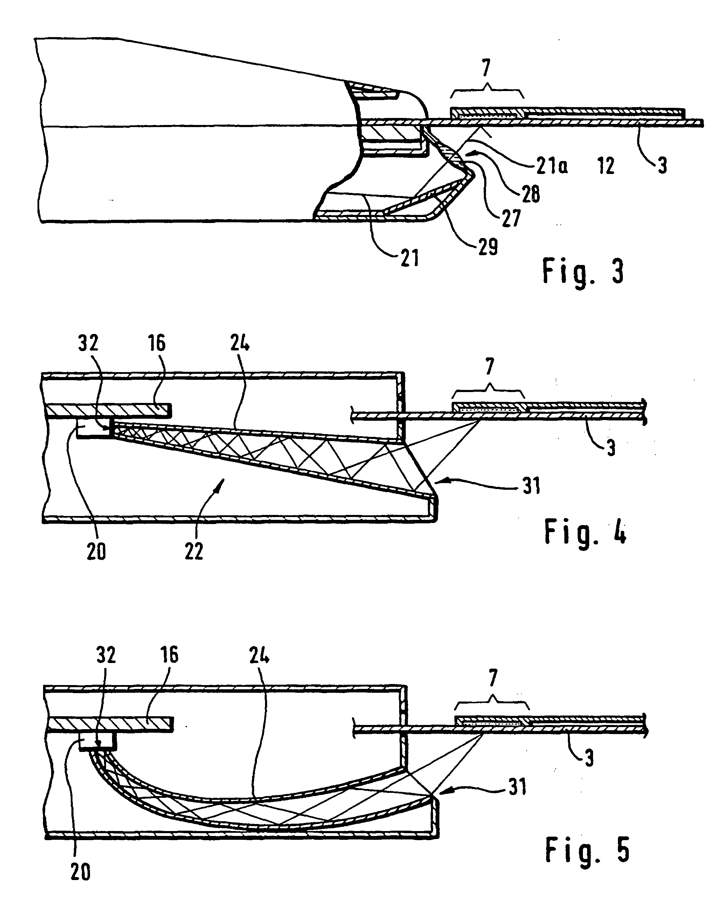

In Figur 3 ist eine alternative Ausgestaltung gezeigt, bei der das optische Abbildungssystem von einer in die Scheibe 28 integrierte optischen Linse gebildet und die erforderliche Strahlumlenkung der IR-Strahlen auf dem Übertragungsweg 21 durch einen Planspiegel 29 gewährleistet wird.

Bei der in den Figuren 2 und 3 dargestellten Ausführungsform basiert die Funktion der ortselektiven Lichttransportmittel 22 weitgehend auf der Wirkung eines optischen Abbildungssystems, das mittels des Abbildungsspiegels 25 oder der Linse 27 realisiert ist. In dem Hohlleiter 24 ist hauptsächlich dessen hintere, schräg geneigte als Planspiegel 30 wirkende Fläche wirksam, die für die erforderliche Umlenkung zu dem IR-Detektor 20 sorgt.

Eine sehr wirksame und dabei besonders kostengünstige Realisierung der ortselektiven Infrarotstrahlen-Transportmittel läßt sich (auch ohne ein optisches Abbildungssystem) mittels eines innenseitig verspiegelten Hohlleiters 24 erreichen, der - wie in den Figuren 4 und 5 dargestellt - so ausgebildet ist, daß seine der Meßzone 7 zugewandte Eingangsöffnung 31 einen größeren Öffnungsquerschnitt als die dem Infrarotdetektor 20 zugewandte Austrittsöffnung 32 hat. Dabei ist es vorteilhaft, wenn sich der Hohlleiter 24 zwischen der Eintrittsöffnung 31 und der Austrittsöffnung 32 im wesentlichen kontinuierlich verjüngt, sein Querschnitt also allmählich immer kleiner wird. Dadurch wird eine Konzentration der Intensität der an den Wänden des Hohlleiters 24 reflektierten Infrarotstrahlung erreicht.

Bei der in Figur 4 dargestellten Ausführungsform verläuft die Achse des Hohlleiters 24 gerade. Die lichtempfindliche Fläche des Detektors 20 befindet sich in diesem Fall seitwärts. Es ist jedoch auch problemlos möglich, den Hohlleiter 24 in einer gekrümmten Form, wie in Figur 5 dargestellt, herzustellen. Eine solche gekrümmte Form ermöglicht eine besonders flexible Gestaltung und Positionierung des Testelementes 3 mit der Testzone 7 und der Leiterplatine 16 mit dem Detektor 20.

Obwohl in den Figuren 4 und 5 kein optisches Abbildungssystem dargestellt ist, besteht selbstverständlich die Möglichkeit, einen Hohlleiter 24 der in diesen Figuren dargestellten Bauart mit einem optischen Abbildungssystem in Form einer Linse oder in Form eines Abbildungsspiegels zu kombinieren.

## Patentansprüche

1. Testelement-Analysesystem (1) zur analytischen Untersuchung einer Probe (8), insbesondere einer Körperflüssigkeit von Menschen oder Tieren umfassend
Testelemente (3) mit einer Meßzone (7), in die die zu untersuchende Probe zur Durchführung einer Analyse gebracht wird, um eine für die Analyse charakteristische Meßgröße zu messen und
ein Auswertegerät (2) mit einer Testelementhalterung (5), um ein Testelement (3) in einer Meßposition zur Durchführung der Messung zu positionieren und einer Meß- und Auswerteelektronik (15) zur Messung der charakteristischen Veränderung und Ermittlung eines hierauf basierenden Analyseresultates,
**dadurch gekennzeichnet, daß**
das Auswertegerät (2) zur Bestimmung der im Bereich der Meßzone (7) des Testelementes (3) herrschenden Temperatur einen Infrarotdetektor (20) aufweist.

2. Analysesystem nach Anspruch 1, **dadurch gekennzeichnet, daß** der Infrarotdetektor (20) in die Meß- und Auswerteelektronik (15) integriert ist.

3. Analysesystem nach einem der Ansprüche 1 oder 2, **dadurch gekennzeichnet, daß** das Auswertegerät (2) Infrarotstrahlen-Transportmittel (22) aufweist, die die Meßzone (7) mit dem Infrarotdetektor (20) ortsselektiv verbinden.

4. Analysesystem nach Anspruch 3, **dadurch gekennzeichnet, daß** die Infrarotstrahlen-Transportmittel (22) einen Hohlleiter (24) mit für Infrarotstrahlen reflektierender Innenwand einschließen.

5. Analysesystem nach Anspruch 4, **dadurch gekennzeichnet, daß** der Hohlleiter (24) aus metallisiertem Kunststoff besteht.

6. Analysesystem nach einem der Ansprüche 4 oder 5, **dadurch gekennzeichnet, daß** die der Meßzone (7) zugewandte Eingangsöffnung (31) des Hohlleiters (24) einen größeren Öffnungsquerschnitt als die dem Infrarotdetektor (20) zugewandte Ausgangsöffnung (32) des Hohlleiters (24) hat.

7. Analysesystem nach einem der Ansprüche 3 bis 6, **dadurch gekennzeichnet, daß** die ortsselektiven Infrarotstrahlen-Transportmittel (22) einen innerhalb des Gehäuses (23) des Auswertegerätes angeordneten Abbildungsspiegel (25) einschließen.

8. Analysesystem nach einem der vorhergehenden Ansprüche, **dadurch gekennzeichnet, daß**
das Testelement (3) in der Meßposition derartig aus dem Gehäuse (23) des Auswertegerätes (2) herausragt, daß die Probe (8) in die Meßzone (7) gebracht werden kann, während sich das Testelement in der Meßposition befindet,
der Detektor (20) in dem Gehäuse (22) positioniert ist,
das Gehäuse (23) ein für Infrarotstrahlen durchlässiges optisches Fenster (26) aufweist und
der Übertragungsweg (21) der Infrarotstrahlen zwischen der Meßzone (7) und dem Infrarotdetektor (20) durch das optische Fenster (26) führt.

9. Analysesystem nach Anspruch 8, **dadurch gekennzeichnet, daß** das optische Fenster (26) mit einer für Infrarotstrahlen durchlässigen Scheibe (28), insbesondere einer Polyethylenfolie, staubdicht verschlossen ist.

10. Analysesystem nach Anspruch 8 in Verbindung mit Anspruch 3, **dadurch gekennzeichnet, daß** die für Infrarotstrahlen durchlässige Scheibe (28) des optischen Fensters (26) mit einer optischen Linse (27) kombiniert ist, die einen Teil der Infrarotstrahlen-Transportmittel (22) bildet.

## Claims

1. Test element analysis system (1) for the analytical investigation of a sample (8), in particular of a body liquid of humans or animals, comprising
test elements (3) with a test zone (7), to which the sample to be analyzed is contacted in order to perform an analysis by measuring a measurement quantity characteristic for the analysis, and
an evaluation instrument (2) with a test element holder (5) for positioning a test element (3) in a measuring position for making the measurement, and with a measurement and evaluation electronics (15) for measuring the characteristic change and deriving an analytical result therefrom,
**characterized in that**
the evaluation instrument (2) comprises an infrared detector (20) for the determination of the temperature in the test zone (7) of the test element (3) .

2. Analysis system according to claim 1, **characterized in that** the infrared detector (20) is integrated into the measuring and evaluation electronics (15).

3. Analysis system according to any one of claims 1 or 2, **characterized in that** the evaluation instrument (2) comprises an infrared radiation transport device (22) for providing a location-selective connection of the test zone (7) with the infrared detector (20).

4. Analysis system according to claim 3, **characterized in that** the infrared radiation transport device (22) comprises a hollow conductor (24) with an interior wall reflective for infrared radiation.

5. Analysis system according to claim 4, **characterized in that** the hollow conductor (24) is made from metal-coated plastic.

6. Analysis system according to any one of claims 4 or 5, **characterized in that** the input opening (31) of the hollow conductor (24) facing the test zone (7) has a larger opening cross-section than the output opening (32) of the hollow conductor (24) facing the infrared detector (20).

7. Analysis system according to any one of claims 3 to 6, **characterized in that** the location-selective infrared radiation transport device (22) includes an imaging mirror (25) located inside the housing (23) of the evaluation instrument.

8. Analysis system according to any one of the preceding claims, **characterized in that**
the test element (3), in the measuring position, sticks out of the housing (23) of the evaluation instrument (2) in such a manner that the sample (8) can be contacted to the test zone (7), while the test element is in the measuring position,
the detector (20) is located in the housing (23),
the housing (23) comprises a window (26) which is transparent for infrared radiation, and
the transport path (21) of the infrared radiation between the test zone (7) and the infrared detector (20) passes through the optical window (26).

9. Analysis system according to claim 8, **characterized in that** the optical window (26) is dust-proof closed by means of a pane (28) transparent to infrared radiation, in particular a polyethylene foil.

10. Analysis system according to claim 8 combined with claim 3, **characterized in that** the infrared transparent radiation pane (28) of the optical window (26) is combined with an optical lens (27) forming a part of the infrared radiation transport device (22).

## Revendications

1. Système d'analyse (1) pour élément d'essai pour l'examen analytique d'un échantillon (8), en particulier un liquide corporel d'hommes ou d'animaux, comprenant
des éléments d'essai (3) avec une zone de mesure (7), dans laquelle on introduit l'échantillon à examiner en vue de réaliser une analyse pour mesurer une grandeur caractéristique de l'analyse, et
un appareil d'évaluation (2) avec un support (5) pour élément d'essai pour positionner un élément d'essai (3) dans une position de mesure permettant d'effectuer la mesure et une électronique de mesure et d'évaluation (15) pour mesurer la variation caractéristique et déterminer un résultat d'analyse basé sur celle-ci,
**caractérisé en ce que**
l'appareil d'évaluation (2), pour déterminer la température régnant au niveau de la zone de mesure (7) de l'élément d'essai (3), est doté d'un détecteur infrarouge (20).

2. Système d'analyse selon la revendication 1, **caractérisé en ce que** le détecteur infrarouge (20) est intégré dans l'électronique de mesure et d'évaluation (15).

3. Système d'analyse selon l'une des revendications 1 ou 2, **caractérisé en ce que** l'appareil d'évaluation (2) est doté de moyens de transport à rayons infrarouges (22) reliant de manière localisée la zone de mesure (7) au détecteur infrarouge (20).

4. Système d'analyse selon la revendication 3, **caractérisé en ce que** les moyens de transport à rayons infrarouges (22) enferment un guide d'ondes (24) muni d'une paroi interne réfléchissant les rayons infrarouges.

5. Système d'analyse selon la revendication 4, **caractérisé en ce que** le guide d'ondes (24) est en matière plastique métallisée.

6. Système d'analyse selon l'une des revendications 4 ou 5, **caractérisé en ce que** l'orifice d'entrée (31) du guide d'ondes (24), orienté vers la zone de mesure (7) a une plus grande section transversale que l'orifice de sortie (32) du guide d'ondes (24), orienté vers le détecteur infrarouge (20).

7. Système d'analyse selon l'une des revendications 3 à 6, **caractérisé en ce que** les moyens de transport à rayons infrarouges (22) localisés enferment un miroir de reproduction (25) disposé à l'intérieur du boîtier (23) de l'appareil d'évaluation.

8. Système d'analyse selon l'une des revendications précédentes, **caractérisé en ce que**
l'élément d'essai (3), dans la position de mesure, déborde du boîtier (23) de l'appareil d'évaluation (2) de telle sorte que l'échantillon (8) puisse être amené dans la zone de mesure (7) alors que l'élément d'essai se trouve dans la position de mesure,
le détecteur (20) est placé dans le boîtier (22),
le boîtier (23) comporte une fenêtre optique (26) perméable aux rayons infrarouges, et
le trajet de transmission (21) des rayons infrarouges entre la zone de mesure (7) et le détecteur infrarouge (20) passe par la fenêtre optique (26).

9. Système d'analyse selon la revendication 8, **caractérisé en ce que** la fenêtre optique (26) est obturée, de manière étanche aux poussières, par une plaque (28) perméable aux rayons infrarouges, en particulier une feuille de polyéthylène.

10. Système d'analyse selon la revendication 8 en combinaison avec la revendication 3, **caractérisé en ce que** la plaque (28) perméable aux rayons infrarouges de la fenêtre optique (26) est combiné avec une lentille optique (27) constituant une partie des moyens de transport à rayons infrarouges (22).
